# EUROPEAN PATENT APPLICATION

(11) **EP 1 229 329 A1**
(43) Date of publication of application: **07.08.2002**
(21) Application number: 00970022.0
(22) Date of filing: 24.10.2000
(51) Int. Cl.: G01N 33/543, G01N 33/53, G01N 33/566, G01N 37/00

(54) **BASES HAVING LIGANDS IMMOBILIZED THEREON**

(30) Priority: 05.11.1999 JP 31561099
(71) Applicant: Takara Shuzo Co, Ltd., Kyoto-shi, Kyoto 612-8061 (JP)
(72) Inventor: KATO, Ikunoshin, Uji-shi, Kyoto 611-0028 (JP); IZU, Hiroyuki, Kusatsu-shi, Shiga 525-0025 (JP); ASADA, Kiyozo, Koka-gun, Shiga 520-3333 (JP)
(74) Representative: Grund, Martin, Dr.
(86) International application number: JP0007415
(87) International publication number: WO0135098

(57) **Abstract**

Bases in the shape of string, tape or circular disc on the surface of which a plural number of different ligands are immobilized respectively in pre-determined domains.

## Description

### Technical Field

The present invention relates to a substrate for successively obtaining at high speed a lot of information on a binding between a ligand and a receptor, an apparatus that utilizes the substrate, an apparatus for detecting a binding between a ligand and a receptor, and a method for detecting the binding, which are useful in a field of biochemistry.

### Background Art

Relationships between structures and activities of molecules are essential matters to be studied with regard to biological systems. Certain molecules are known to interact with and bind to other molecules. Such specific intermolecular bindings are known as relationships between receptors and ligands. A number of methods are known for determining binding affinities between ligands and receptors (see, for example, JP-A 4-505763).

### Objects of Invention

The main object of the present invention is to provide a cord-, tape- or disk-shaped substrate having various ligands being immobilized for successively analyzing at high speed information on bindings between various ligands and receptors as well as an apparatus that utilizes the substrate.

### Summary of Invention

The present invention is outlined as follows. The first aspect of the present invention relates to a cord-, tape- or disk-shaped substrate having plural different ligands being immobilized in predetermined regions on its surface.

In one embodiment, the ligand is exemplified by a nucleic acid, a saccharide, a peptide or a protein. The nucleic acid is exemplified by a double-stranded DNA or a single-stranded DNA, and the DNA is exemplified by a polynucleotide or an oligonucleotide. The substrate is preferably one that can be used to detect at high speed a binding between a ligand and a receptor having an ability to bind to the ligand. An example of the substrate of the present invention is one in which the distance between the regions on the substrate is at least 1 mm, the density of the regions on the substrate is less than 100 regions/cm², the density of the regions on the substrate is less than 10 regions/cm², the number of the regions on the substrate is 100 or more, or the number of the regions on the substrate is 1000 or more. The receptor is exemplified by a nucleic acid, a saccharide, a peptide or a protein that has an ability to bind to a nucleic acid, a saccharide, a peptide or a protein. A receptor with a label can be used as the receptor.

The second aspect of the present invention relates to apparatus for detecting a binding between a ligand and a receptor, which comprises:
a) a cord-, tape- or disk-shaped substrate having plural different ligands being immobilized in predetermined regions on its surface;
b) a means of exposing the substrate to a receptor that binds to at least one of the ligands; and
c) a means of detecting a region of a binding between the receptor and the at least one of ligands on the substrate.

The third aspect of the present invention relates to an apparatus for detecting a binding between a ligand and a receptor, which comprises a means of detecting at high speed a region of a binding between a receptor and one of plural different ligands on a cord-, tape- or disk-shaped substrate having the ligands being immobilized in predetermined regions on its surface.

The fourth aspect of the present invention relates to a method for detecting a binding between a ligand and a receptor, the method comprising:
a) exposing a cord-, tape- or disk-shaped substrate having plural different ligands being immobilized in predetermined regions on its surface to a receptor that binds to at least one of the ligands; and
b) detecting a region of a binding between the receptor and the at least one of ligands on the substrate.

In the second to fourth aspects, it is preferable to use the substrate of the first aspect. Using a receptor with a label, the region of a binding between the receptor and the ligand on the substrate can be detected by means of the label. As a receptor, it is preferable to use a nucleic acid, a saccharide, a peptide or a protein that has an ability to bind to a nucleic acid, a saccharide, a peptide or a protein as a ligand.

### Brief Description of Drawings

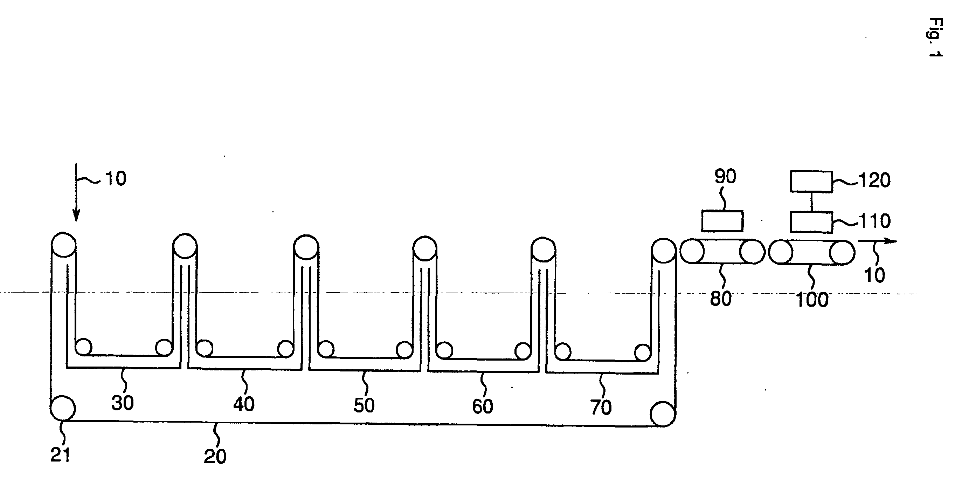

Figure 1 is a schematic diagram that illustrates an exemplary detection apparatus according to the present invention.

### Detailed Description of the Invention

There is no specific limitation concerning a ligand according to the present invention as long as it is a molecule recognized and bound by a specific receptor. For example, the ligand is a nucleic acid, a saccharide, a protein or a peptide.

Examples of ligands include agonists and antagonists of cell membrane receptors, toxins and venoms, epitopes of viruses, hormones (e.g., sedatives, opiates and steroids), hormone receptors, peptides, enzymes, substrates for enzymes, cofactors, drugs, lectins, saccharides, oligonucleotides, polynucleotides, nucleic acids, oligosaccharides, proteins as well as monoclonal antibodies.

There is no specific limitation concerning a receptor according to the present invention as long as it has an ability to bind to the above-mentioned ligand. It may be a naturally occurring molecule or an artificial molecule. Examples of receptors include nucleic acids, saccharides, proteins and peptides such as enzymes, cell surface proteins (receptors), glycoproteins and antibodies.

The properties of the contacting surfaces of a ligand and a receptor having an ability to bind to the ligand according to the present invention are complementary each other.

As used herein, the term "cord-shaped" means that the width of a substance is narrower than that of a tape-shaped one, and encompasses cord-shaped, string-shaped and rope-shaped. A cord-shaped substrate may be made from any material as long as ligands can be immobilized in predetermined regions on the surface of the substrate. A tape-shaped substrate is a narrow, long and belt-like substrate. A tape-shaped substrate may be made from any material as long as ligands can be immobilized in predetermined regions on the surface of the substrate. A disk-shaped substrate according to the present invention is a disk-shaped substrate that may be made from any material as long as ligands can be immobilized in predetermined regions on the surface of the substrate.

Any flexible or semi-flexible material may be used for a cord-shaped substrate. It is desirable that plural ligand immobilization regions are placed at physically separated positions on the surface. Any flexible or semi-flexible material may be used for a tape-shaped substrate. At least one surface of the tape-shaped substrate is substantially flat. It is desirable that plural ligand immobilization regions are placed at physically separated positions on the surface. Any rigid, flexible or semi-flexible material may be preferably used for a disk-shaped substrate. At least one surface of the disk-shaped substrate is substantially flat. It is desirable that plural ligand immobilization regions are placed at physically separated positions on the surface.

There is no specific limitation concerning the array of ligand immobilization sites on the surface of a cord-, tape- or disk-shaped substrate. The array may be changed depending on the purpose.

For example, ligands may be spotted at density of 1 to 100 ligands/cm, 1 to 100 ligands/cm², or 1 to 100 ligands/cm³ onto a cord-shaped substrate. Ligands may be spotted at density of 1 to 100 ligands/cm along the short side and 1 to 100 ligands/cm along the long side onto a tape-shaped substrate. In case of a disk-shaped substrate, ligands may be spotted at density of 1 to 10000 ligands/cm².

Ligands may be conveniently spotted onto a substrate using a commercially available spotter. Certain types of ligands can be synthesized on a substrate.

A substrate that can be used to detect at high speed a binding between a ligand and a receptor having an ability to bind to the ligand is preferably used as a substrate according to the present invention. When a binding between a ligand and a receptor having an ability to bind to the ligand is detected at high speed using such a substrate, a number of bindings between ligands and receptors having abilities to bind to the ligands can be detected in a short time even if a substrate in which ligand immobilization regions are positioned at a distance each other is used. A substrate in which ligand immobilization regions are positioned at a distance each other can be conveniently prepared. Even if such a substrate is used, effects equivalent to those accomplished by using a substrate having ligand immobilization regions at high density can be brought by obtaining information from the substrate at high speed.

Furthermore, chance of contamination among ligands upon preparation of can be reduced for a substrate in which ligand immobilization regions are positioned at a distance each.

The substrates are exemplified by the following, all of which can be preferably used according to the present invention: a cord-, tape- or disk-shaped substrate in which ligand immobilization regions are positioned at intervals of at least 1 mm; a cord-, tape- or disk-shaped substrate in which ligand immobilization regions are positioned at density of less than 100 regions/cm²; a cord-, tape- or disk-shaped substrate in which ligand immobilization regions are positioned at density of less than 10 regions/cm²; a cord-, tape- or disk-shaped substrate in which the number of ligand immobilization regions on the substrate is 100 or more; and a cord-, tape- or disk-shaped substrate in which the number of ligand immobilization regions on the substrate is 1000 or more.

A binding between a ligand and a receptor of interest can be detected only by one-dimensional scanning if ligand immobilization regions are positioned in a specific area in a line according to the present invention. On the other hand, two-dimensional scanning is required in order to detect a binding between a ligand and a receptor of interest using a conventional substrate which contains ligand immobilization regions at high density. Thus, the present invention provides an epochally convenient method for detecting a binding between a ligand and a receptor of interest as compared with conventional ones.

The line as mentioned above is not limited to a straight line or a curve.

A substrate made from a substantially insoluble material can be preferably used as the cord- or tape-shaped substrate of the present invention. Examples of such materials include hetero-polymers, polyurethane, polyester, polycarbonate, polyurea, polyamide, polyethylene imine, polyarylene sulfide, polysiloxane, polyimide, polyacetate, polyvinyl chloride, nylon and other polymers which would become known in the art after the disclosure of the present application, as well as paper, nylon, cellulose and cellulose derivatives such as nitrocellulose, to which ligands are covalently or non-covalently attached.

A substrate made from a substantially insoluble material can be preferably used as the disk-shaped substrate of the present invention. Examples of such materials include hetero-polymers, polyurethane, polyester, polycarbonate, polyurea, polyamide, polyethylene imine, polyarylene sulfide, polysiloxane, polyimide, polyacetate, polyvinyl chloride, nylon and other polymers which would become known in the art after the disclosure of the present application, paper, nylon, cellulose, cellulose derivatives such as nitrocellulose, plastics such as polycarbonate, polystyrene and polypropylene as well as plastic derivatives, magnetic or nonmagnetic metals, quartz and glass, to which ligands are covalently or non-covalently attached.

The cord-, tape- or disk-shaped substrate of the present invention may be made from a polymeric or non-polymeric, porous material having a smooth surface. However, the substrate used according to the present invention is not limited to one made from such a material. For example, the substrate may be one that can be used as a substrate for a DNA chip or a biosensor and that can retain DNAs to be immobilized on its surface. For example, a substrate having a hydrophilic or hydrophobic functional group on its surface can be preferably used. Examples of functional groups include a hydroxyl group, an amine group, a thiol group, an aldehyde group, a carboxyl group and an acyl group. The substrates also include a substrate having such a functional group on its surface as a property of the material of the substrate. Even if a substrate does not have a hydrophilic or hydrophobic functional group on its surface, it may be used without limitation as long as it can be made to have such a functional group on its surface by surface treatment.

Examples of substrates subjected to such surface treatment include a substrate having a surface treated with a commercially available silane-coupling agent such as aminoalkylsilane, with a polycation such as polylysine or polyethylenimine, or with aminoalkylsilane and glutaraldehyde.

An electrically negative, neutral or positive substrate may be preferably used as the cord-, tape- or disk-shaped substrate of the present invention if ligands are efficiently immobilized onto it. Furthermore, the substrate may have a dual structure consisting of a part for ligand immobilization and a part for supporting the portion for ligand immobilization. It may be selected considering ligand immobilization efficiency and strength of the cord-, tape- or disk-shaped substrate.

A part for ligand immobilization may be prepared beforehand, stuck to a part for supporting the part for ligand immobilization, and then used. There is no limitation concerning the shape of the part for ligand immobilization. Cord-, tape- or disk-shaped substrates may be prepared using beads onto which ligands have been immobilized beforehand, beads on which ligands are synthesized or the like.

Any material may be preferably used for the cord-, tape- or disk-shaped substrate without limitation as long as the background is repressed sufficiently for detecting the signals using a detector. A transparent, semitransparent or opaque substrate may be preferably used according to the present invention. A light transmission-type detector, a light reflection-type detector or the like may be selected and used depending on the material of the substrate.

The substrate may be one-dimensionally moved for detection using a detector. Alternatively, the detector may be one-dimensionally moved on the substrate.

A binding between a ligand and a receptor therefor can be determined by exposing a cord-, tape- or disk-shaped substrate having plural ligands to at least one receptor (for example, a ligand with a label) of which the ability to bind to the ligands is to be determined, and detecting a position of the label on the substrate. The whole substrate may be exposed to the receptor or portions of the substrate may be successively exposed to the receptor. For example, a binding between a ligand and a receptor can be successively determined as follows: in case of a cord-shaped substrate, exposure to a receptor may be started from the tip of the substrate; in case of a tape-shaped substrate, exposure to a receptor with a label may be started from one short side, and the position of the label on the substrate is then detected; and in case of a disk-shaped substrate, exposure to a receptor with label may be started from any position on the substrate, and the position of the label on the substrate is then detected. Optionally, a step of washing and/or drying the cord-, tape- or disk-shaped substrate may be appropriately included in the above-mentioned procedure.

The speed of passage of a cord-, tape- or disk-shaped substrate through a detector for the substrate may be appropriately selected upon determination of a binding between a ligand and a receptor. For example, the speed may be constant. Alternatively, the speed may be changed for regions to be precisely examined. If a substrate is passed at constant speed, the speed for a resolution of 10 µm is, for example, preferably 15 seconds/cm² or less, more preferably 10 seconds/cm² or less. The speed for a resolution of 80 µm is preferably 1 second/cm² or less, more preferably 0.5 second/cm² or less. Detection may be carried out by repeating the passage of the whole cord-, tape- or disk-shaped substrate, or regions thereof to be precisely examined, through a detector while reversing the transfer direction of the substrate such that the signal data is integrated.

A step of detecting a binding of a ligand and a receptor can be carried out at high speed by using a substrate having been subjected to a step of binding the receptor to the ligand beforehand. In this case, even if a substrate having ligand immobilization regions at low density is used, effects equivalent to those accomplished by using a substrate having ligand immobilization regions at high density can be brought as a result of the high speed. Furthermore, since ligands can be immobilized onto a low-density substrate precisely, conveniently and at low cost, the substrate of the present invention has industrial utility equivalent to or greater than that of a substrate having ligand immobilization regions at high density.

In one preferred embodiment, linker molecules are conferred on a substrate. Ligands are reacted with functional groups exposed on the linker molecules. In a preferred embodiment, ligands have amino groups or carboxyl groups at their termini, and linker molecules have amino groups or carboxyl groups that are reactive with the termini of the ligands.

The resulting substrate has various uses including, for example, screening of a number of receptors for a biological activity. The substrate is exposed to one or more receptors in order to screen for a biological activity. The receptor may be, for example, one selected from antibodies, whole cells, receptors on vesicles, lipids or other various receptors. The receptor is preferably labeled, for example, with a fluorescent label, a radioactive label or a labeled antibody reactive with the receptor. For example, the position of the label on the substrate is detected by a photon detection method or an autoradiography method. Although it is not intended to limit the present invention, for example, a single fluorescent label may be fused for detection. In this case, existence of a receptor can be compared and analyzed among test samples by reacting plural test samples having a single fluorescent label with plural cord-, tape- or disk-shaped substrates of the present invention separately, and then detecting fluorescent signals on the plural cord-, tape- or disk-shaped substrates simultaneously or separately at high speed. The analysis may be carried out using two or more fluorescent labels.

Furthermore, a binding between a receptor and a ligand immobilized onto a substrate can be detected by detecting a magnetic field of a magnetic material used to label the receptor.

Thus, the present invention provides a method for detecting a binding between a ligand and a receptor in which a binding between a receptor and a ligand immobilized in a predetermined region on a substrate is detected by detecting a magnetic field of a magnetic material used to label the receptor.

For example, in such a method, a receptor is labeled with a demagnetized magnetic microparticle bound to or coated with a linker molecule having a functional group that binds to the receptor.

After the receptor labeled with the demagnetized magnetic microparticle is exposed to ligands, the receptor unbound to the ligands is washed and removed.

The magnetic microparticle attached to the receptor being bound to the ligands is re-magnetized by exposing it to a magnetic field at an appropriate level.

As a result, a binding between a ligand and a receptor can be detected by detecting the magnetic field of the re-magnetized magnetic microparticle.

A hard magnetic material can be used for a magnetic microparticle according to the present invention. Although it is not intended to limit the present invention, magnetic powder prepared by growing microparticles of magnetite (Fe₃O₄) or maghemite (gamma Fe₂O₃) to form a needle-shaped substance can be used as a hard magnetic material. Highly precise detection can be accomplished by using a hard magnetic material having a high magnetic saturation point or a hard magnetic material that is not subject to heat demagnetization. Optionally, a receptor having a magnetic material attached through a hydrophobic bond may be used. A magnetic material may be used without demagnetization. A magnetic material derived from a microorganism may be used.

A ligand that binds to a receptor can be rapidly determined through information on the ligand at the position at which a binding is detected. Therefore, a number of receptors can be screened using this technique. Other possible uses of the present invention include diagnosis. In this case, various ligands for specific receptors are placed on a substrate, and diseases associated with abnormalities of receptors are diagnosed based on the presence of the receptors in test samples.

Determination of a binding between a ligand and a receptor can be automated by using an apparatus for determining a binding between a ligand and a receptor of the second aspect of the present invention. The apparatus is one for detecting a binding between a ligand on a cord-, tape- or disk-shaped substrate having plural different ligands being immobilized in predetermined regions on its surface and a receptor in a test sample and comprises:
a) a means of exposing the substrate to a receptor that binds to at least one of the ligands; and
b) a means of detecting a region of a binding between the receptor and the at least one of ligands on the substrate.

For example, the apparatus may be composed of a means of transferring a cord-, tape- or disk-shaped substrate, a pre-prehybridization bath, a prehybridization bath, a labeled receptor bath, a washing bath, a means of drying, a means of detecting a binding between a ligand and a receptor, a computer for analyzing data on a binding between a ligand and a receptor. A single bath or plural baths may serve as the respective baths.

Figure 1 illustrates the structure of an exemplary detection apparatus of the present invention. A tape-shaped substrate having ligands being immobilized (10) is transferred to a pre-prehybridization bath (30) for pretreatment with the aid of a means of transferring a tape-shaped substrate (20), which is driven by a roller (21). The pretreated substrate is transferred to a prehybridization bath (40) followed by a labeled receptor bath (50). A binding between a ligand and a receptor occurs in the labeled receptor bath. The substrate is washed in a washing bath I (60) followed by a washing bath II (70). After washing, the substrate is transferred with the aid of a means of transferring for drying (80) and dried using a means of drying (90). The substrate is then transferred with the aid of a means of transferring for detection of a binding between a ligand and a receptor (100). A binding between a ligand and a receptor is detected using a means of detecting a binding between a ligand and a receptor (110). The information on the detected binding is analyzed using a computer for analyzing data on a binding between a ligand and a receptor (120).

A cassette is exemplified as an apparatus in which a single bath serves as the respective baths. The cassette contains a combination of a cord- or tape-shaped substrate and a means of winding, or a combination of a disk-shaped substrate and a means of rotating, in a container that functions as a bath and has an inlet and an outlet. In this case, for example, the procedure from the step of pretreatment to the step of washing is carried out in the cassette while exchanging the fluid. Then, the cassette is equipped with a means of drying/detection to dry the substrate and detect a binding between a ligand and a receptor while transferring the substrate with the aid of the means of winding or rotating.

Furthermore, determination of a binding between a ligand and a receptor can be automated by using an apparatus for determining a binding between a ligand and a receptor of the third aspect of the present invention. The apparatus is one for detecting a binding between a ligand on a cord-, tape- or disk-shaped substrate having plural different ligands being immobilized in predetermined regions on its surface and a receptor in a test sample and comprises:
a means of detecting a region of a binding between a receptor and a ligand on the substrate.

If such an apparatus is used, it is convenient to use a substrate having been subjected to a step of binding a receptor to ligands on the substrate beforehand.

After binding a receptor to ligands on a substrate, a cord-, tape- or disk-shaped substrate may be prepared to include the regions at which the ligands are immobilized. The regions are then detected using the apparatus.

The time required for measurement using the apparatus can be regulated by adjusting the speed of transfer of the cord-, tape- or disk-shaped substrate in the apparatus. Thus, a binding between a ligand and a receptor can be determined at higher speed.

The present application discloses an improved detection apparatus and an improved method for detection. A cord-, tape- or disk-shaped substrate having plural ligands being immobilized at known positions on its surface is used for the method and apparatus for detection. The cord-, tape- or disk-shaped substrate is exposed to a receptor (e.g., a receptor with a fluorescent label), for example, in a successive manner. The receptor binds to one or some of the ligands. If several sets of a group of ligands as a unit are immobilized on a cord-, tape- or disk-shaped substrate in a repeated manner, each group of the ligands as a unit may be exposed to each of plural receptors. In other words, if the apparatus has plural labeled receptor baths, each group as a unit can be exposed to each labeled receptor. The cord-, tape- or disk-shaped substrate is then successively placed in a detector (e.g., a microscopic detector) for identifying the position of a binding. A microscopic detector comprises a light source for directing light to a substrate, a means of detecting, for example, fluorescence from a substrate, and a means of determining the position of fluorescence. A detector that detects a binding between a ligand and a receptor as electricity in time course may be used. The ratio of signal to background (signal to noise ratio) for the detected signal can be improved by subjecting the cord-, tape- or disk-shaped substrate to several rounds of measurements to integrate the signal.

Examples of means of detecting fluorescence on the cord-, tape- or disk-shaped substrate include a photon counter. A means of determining the position of a binding between a ligand and a receptor may be fixed such that the cord-, tape- or disk-shaped substrate is passed through it. Alternatively, it may be capable of X-Y movement. Data collected from the cord-, tape- or disk-shaped substrate passed through the detector is recorded using an appropriately programmed digital computer, and then analyzed.

### Examples

The following Examples further illustrate the present invention in detail but are not to be construed to limit the scope thereof.

### Example 1

(1) Examination was carried out for genes influenced by endocrine disruptors. Specifically, 33 genes were selected from a group of genes that are potentially influenced by endocrine disruptors. The genes are shown in Table 1.

**Table 1**

| Gene no. | Name of immobilized gene (Gene product name) | Combination of primers (SEQ ID NO) |
|---|---|---|
| 1 | Smad3 | 1, 2 |
| 2 | VEGF receptor | 3, 4 |
| 3 | ACTR | 5, 6 |
| 4 | N-CoR/SMRT | 7, 8 |
| 5 | efp | 9,10 |
| 6 | c-Myc-1 | 11,12 |
| 7 | Vitamin D receptor | 13,14 |
| 8 | cathepsin G | CAP * |
| 9 | c-Myc-2 | 15,16 |
| 10 | Bax | 17,18 |
| 11 | JNK1 | 19,20 |
| 12 | p38 | 21,22 |
| 13 | TRIP 1 | 23,24 |
| 14 | ARA 70 | 25,26 |
| 15 | insulin receptor | 27,28 |
| 16 | NGF receptor | CAP * |
| 17 | PDGF receptor | 29,30 |
| 18 | CSF1 receptor-1 | CAP * |
| 19 | CSF1 receptor-2 | 31,32 |
| 20 | FGF receptor | 33,34 |
| 21 | p38 gamma | 35,36 |
| 22 | Bcl-X | 37,38 |
| 23 | c-Myc-3 | 39,40 |
| 24 | pS2 protein | 41,42 |
| 25 | lactoferrin | 43,44 |
| 26 | RIP 140 | 45,46 |
| 27 | TIF2 | 47,48 |
| 28 | JNK2 | 49,50 |
| 29 | Bax delta | 51,52 |
| 30 | BMK-1 | 53,54 |
| 31 | BMK-2 | 55,56 |
| 32 | Src-1 | 57,58 |
| 33 | p300/CBP | 59,60 |
| 34 | β-actin (positive control) | 61,62 |
| 35 | pBR 322 (negative control) | |

| | | |
|---|---|---|
| * CAP: commercially available primers. | | |

Genes selected from those listed in Table 1 or DNA fragments thereof were prepared as follows. Information on nucleotide sequences of genes, or genes for gene products, to be immobilized, which are submitted under specific accession numbers, was obtained from GenBank. Primer pairs for PCR amplification were constructed based on the information. OLIGO™ Primer Analysis Software (Takara Shuzo) was used for the construction setting the parameters of the software to result in DNA fragments of about 100 b to about 1 kb, which are optimal for the method of the present invention. Primers were then synthesized using a DNA synthesizer according to the determined nucleotide sequences. For cathepsin G, NGF receptor and CSF1 receptor genes, primers for the respective genes contained in Human UniGene DNA set (Research Genetics) were used.

PCR amplification fragments of interest were obtained according to a standard protocol attached to a commercially available PCR kit using the primer pairs as well as a genomic DNA, a genomic DNA library or a cDNA library as a template. The thus obtained DNA fragments were purified using SUPREC-02 (Takara Shuzo). Nucleotide sequences of the amplified cDNAs were analyzed to determine if they were the fragments of interest. Each of the amplified fragments was recovered by ethanol precipitation and dissolved in 100 mM carbonate buffer (pH 9.5) at a concentration of 1 µM. In addition, a housekeeping gene, β-actin gene, as a positive control was prepared in a similar manner. A plasmid pBR322 DNA was used as a negative control.

Five serial dilutions containing were prepared for each of the purified DNA fragments after dissolving a portion of each purified DNA fragment in 50 mM carbonate buffer (pH 9.5) and measuring the absorbance at 260 nm to determine the concentration. Each dilution contained the DNA fragment at concentrations ranging from 0.1 to 0.5 mg/ml. Samples to be used for alkali denaturation as described below were prepared such that the treatment would result in the concentrations as described above.

The respective samples were allowed to stand at room temperature for 10 minutes as they were or after adding them to 0.25 N sodium hydroxide containing 0.5 M sodium chloride (Nacalai Tesque), and then cooled on ice bath. The DNA solutions were spotted onto a cord-, tape- or disk-shaped substrate using GMS 417 Arrayer (Genetic Microsystems, GMS). Cord-shaped substrates were prepared by cutting one of two types of nylon membranes, Hybond-N (Amersham) and Gene Screen Plus (NEN), into strips of 1 mm wide and 1 m long, and twisting the strips into cords. The samples were spotted onto the cords at intervals of 1 mm or longer to result in spots of 0.1 mm in diameter. Two groups of 5 serial dilutions of 35 DNA fragments with alkali denaturation (35 x 5 = 175 spots) or without alkali denaturation (35 x 5 = 175 spots) were spotted in a repeated manner. Two types of nylon membranes, Hybond-N (Amersham) and Gene Screen Plus (NEN), were used as tape-shaped substrates. The membranes were cut into tapes of 1 cm wide and 1 m long to prepare tape-shaped substrates. Samples were spotted onto the tapes to form arrays of spots (0.1 mm in diameter). The array comprised 5 columns and 700 rows (4 groups of 175 rows). Specifically, sets of two groups of 5 serial dilutions of 35 DNA fragments with alkali denaturation (35 x 5 = 175 spots) or without alkali denaturation (35 x 5 = 175 spots) were spotted in a repeated manner. On the other hand, in case of a disk-shaped substrate, two groups of 5 serial dilutions of 35 DNA fragments with alkali denaturation (35 x 5 = 175 spots) or without alkali denaturation (35 x 5 = 175 spots) were spotted in a repeated manner onto a ϕ 87-mm disk-shaped nylon membrane, Hybond-N (Amersham).

Each cord-, tape- or disk-shaped substrate onto which the DNA fragments were spotted as described above was allowed to stand at room temperature for 30 minutes, washed in 0.5 M tris-hydrochloride (pH 7.5) containing 0.5 M sodium chloride and then rinsed in 2 x SSC (Wako Pure Chemical Industries). The substrate was subjected to UV radiation for about 2 minutes using a transilluminator (Cosmo Bio) to immobilize the DNAs. Thus, DNA arrays on cord-, tape- or disk-shaped substrates were prepared.

(2) Influences of various endocrine disruptors on cultured cells were examined.

Human breast cancer MCF-7 cells were grown in DME medium containing 10% fetal bovine serum (FBS). After trypsinization, 2 x 10⁶ cells were placed in a 10-cm dish. The cells were cultured for 24 hours in DME medium containing 5% fetal bovine serum from which steroid hormones had been removed by treatment with activated carbon-dextran. After the medium was removed, the cells were cultured for 2 hours in the same medium containing diethylstilbestrol (DES) at a concentration of 10 nM. Cells cultured in a similar manner in the absence of the chemical substance were prepared as a control. Total RNAs were prepared using TRIzol reagent (Gibco-BRL) from the respective cells recovered after treatments.

(3) The total RNA prepared in (2) above was treated with DNase. 12 µl of a reaction mixture containing about 100 to about 300 µg of the total RNA, 10 µl of 10 x AMV buffer (Life Science) and 10 U of DNaseI (Takara Shuzo) was prepared, incubated at 37°C for 10 minutes, and subjected to two rounds of phenol-chloroform extraction followed by ethanol precipitation. The concentration was determined using a portion of the resulting total RNA.

(4) A reverse transcription reaction was carried out using the total RNA prepared in (3) above. The composition of the reaction mixture is shown below:
Reaction mixture A: about 130 µg of the total RNA, 10 µg of an oligo(dT) primer (Takara Shuzo) and 20 µl of water treated with diethylpyrocarbonate (DEPC, Wako Pure Chemical Industries); and
Reaction mixture B: 12 µl of 5 x AMV RTase buffer (Life Science), 0.5 mM each of dATP, dCTP and dGTP, 0.2 mM of dTTP, 60 U of RNase inhibitor (Takara Shuzo), 0.1 mM of Cy3-labeled dUTP (Amersham Pharmacia).

The reaction mixture A was incubated at 70°C for 10 minutes, and then cooled on ice bath. The reaction mixture B was added thereto, and the resulting mixture was incubated at 42°C for 5 minutes. About 60 U of AMV RTase (Life Science) was further added thereto, resulting in a total reaction volume of 60 µl. The RT reaction mixture was incubated at 42°C for 70 minutes. 7.5 µl of 500 mM EDTA solution and 15 µl of 1 M sodium hydroxide were added to the reaction mixture. The resulting mixture was incubated at 60°C for 1 hour to degrade the template RNA. After cooling to room temperature, 37.5 µl of 1 M tris-hydrochloride (pH 7.5) was added thereto. The resulting solution was concentrated using Microcon™-30 (Millipore) to a volume of 20 µl. After adding 200 µl of 10 mM tris-hydrochloride containing 1 mM EDTA thereto, the mixture was concentrated to a volume of 20 µl again. This Cy3-labeled DNA solution was used for hybridization below.

(5) The cord-, tape- or disk-shaped substrate prepared as described in (1) above was subjected to permeation with 2 x SSC for 1 minute, and then subjected to prehybridization in a solution containing 4 x SSC, 0.2% SDS and 100 µg/ml salmon sperm DNA at 40°C for 1 hour. Next, the DNA array on the cord-, tape- or disk-shaped substrate was incubated at 40°C overnight in a hybridization solution. The hybridization solution was prepared by heat-denaturing the Cy3-labeled target DNA prepared in (4) above and suspending it in a solution containing 4 x SSC, 0.2% SDS and 100 µg/ml salmon sperm DNA such that the concentration of the heat-denatured target DNA became about 10 µg/ml. After hybridization, the substrate was washed twice in a solution containing 2 x SSC and 0.1% SDS at room temperature for 5 minutes, followed by twice in solution containing 0.1 x SSC and 0.1% SDS at 68°C for 15 minutes. Fluorescent signals from the respective spots on the substrate were measured as follows. The substrate was subjected to a fluorescence detector. The detector can be used to measure in a successive manner from one short side (in case of the cord- or tape-shaped substrate) or from any measurement start point (in case of the disk-shaped substrate). Bindings between the labeled receptor and the ligands were then analyzed. The results of analysis of the array on the substrate were numerically expressed by dividing the fluorescence signal value for the sample treated with the endocrine disruptor by the fluorescence signal value for the control. If the calculated value is greater than 1.00, the expression of the gene is enhanced by the treatment with the endocrine disruptor. On the other hand, if the value is smaller than 1.00, the expression is suppressed by the treatment. If the value is equal to 1.00, the gene is not influenced by the treatment with the substance.

According to the above-mentioned analysis, the analytical values for N-COR/SMRT and ARA 70 (nuclear receptors and nuclear receptor transcriptional coupling factors), p38r, JNK2 and BMK2 (kinase-type signal transduction factors), and PDGF receptor (receptor-type kinases) were smaller than 1.00. Thus, it was confirmed that the expression of these genes were suppressed in response to the stimuli with DES which exhibits an endocrine-disrupting activity. Furthermore, it was confirmed that denaturation into single strands prior to spotting resulted in higher signal intensities. In addition, similar results were obtained using the two nylon membranes, Hybond-N and Gene Screen plus according to the present invention. As described above, significant change in a signal corresponding to expression of a gene that is potentially influenced by an endocrine disruptor could be observed using the cord-, tape- or disk-shaped substrate of the present invention. Thus, a gene that is influenced by an endocrine disruptor could be detected and identified.

### Example 2

(1) An apparatus composed of a means of transferring a cord- or tape-shaped substrate, a pre-prehybridization bath, a prehybridization bath, a labeled receptor bath, a washing bath I, a washing bath II, a means of drying, a means of detecting a binding between a ligand and a receptor, as well as a computer for analyzing data on a binding between a ligand and a receptor was prepared. Bindings between ligands and a receptor were successively determined using the apparatus by successively transferring the cord- or tape-shaped'substrate prepared in Example 1(1). The pre-prehybridization bath contained a 2 x SSC solution. The prehybridization bath contained a solution containing 4 x SSC, 0.2% SDS and 100 µg/ml salmon sperm DNA. The labeled receptor bath contained a hybridization solution. The hybridization solution was prepared by heat-denaturing the Cy3-labeled target DNA prepared in Example 1(4) and suspending it in a solution containing 4 x SSC, 0.2% SDS and 100 µg/ml salmon sperm DNA such that the concentration of the heat-denatured target DNA became about 10 µg/ml. The washing bath I contained a solution containing 2 x SSC and 0.1% SDS. The washing bath II contained a solution containing 0.1 x SSC and 0.1% SDS.
(2) The tape-shaped substrate prepared in Example 1(1) was set in the apparatus, and passed through the pre-prehybridization bath at room temperature from one short side. It was placed in the prehybridization bath at 40°C and incubated for 1 hour. The substrate was then placed in the labeled receptor bath which contained Cy3-labeled target DNA (receptor) at 40°C and incubated overnight. The substrate was washed by passing it through the washing bath I at room temperature, followed by the washing bath II at 68°C. After washing, the tape-shaped substrate was subjected to a fluorescence detector to measure fluorescent signals from the respective spots. Bindings between the labeled receptor and the ligands were then analyzed. In consequence, results similar to those obtained in Example 1 were obtained.
(3) The procedure as described in (2) above was carried out using the disk-shaped substrate prepared in Example 1(1) in an apparatus. The apparatus was similar to the one as described in (1) above except that it had a means of transferring a disk-shaped substrate in place of the means of transferring a cord- or tape-shaped substrate. Fluorescent signals from the respective spots on the disk-shaped substrate were measured. Bindings between the labeled receptor and the ligands were then analyzed. In consequence, results similar to those obtained in Example 1 were obtained.

### Industrial Applicability

The present invention provides a substrate and measurement apparatus for successively determining at high speed a binding between a ligand and a receptor. The present invention provides a cord-, tape- or disk-shaped substrate having nucleic acids, saccharides, proteins or peptides being immobilized, which is useful in various fields of biochemistry, diagnosis and the like. Furthermore, a binding between a ligand and a receptor can be determined at high speed by using the cord-, tape- or disk-shaped substrate without increasing the density of ligands in a unit area on the substrate. Thus, the present invention enables detection of a binding between a ligand and a receptor at high speed. The high-speed detection can bring effects as if the density on the substrate are increased. Thus, effects equivalent to or more than those accomplished by using a high-density substrate can be brought using a substrate having ligand immobilization regions at low density. Such a substrate can be prepared at low cost, precisely and conveniently. Furthermore, since ligands are positioned in a line according to the present invention, a detector only has to detect the presence of a binding between a ligand and a receptor, and there is no need to conduct secondary scanning upon detection of a labeled substance using the detector. The present invention is also remarkably convenient at this point.

### Sequence Listing Free Text

SEQ ID NO: 1: Designed oligonucleotide primer for amplifying a portion of Smad3 gene.
SEQ ID NO: 2: Designed oligonucleotide primer for amplifying a portion of Smad3 gene.
SEQ ID NO: 3: Designed oligonucleotide primer for amplifying a portion of VEGF receptor gene.
SEQ ID NO: 4: Designed oligonucleotide primer for amplifying a portion of VEGF receptor gene.
SEQ ID NO: 5: Designed oligonucleotide primer for amplifying a portion of ACTR gene.
SEQ ID NO: 6: Designed oligonucleotide primer for amplifying a portion of ACTR gene.
SEQ ID NO: 7: Designed oligonucleotide primer for amplifying a portion of N-CoR/SMRT gene.
SEQ ID NO: 8: Designed oligonucleotide primer for amplifying a portion of N-CoR/SMRT gene.
SEQ ID NO: 9: Designed oligonucleotide primer for amplifying a portion of efp gene.
SEQ ID NO: 10: Designed oligonucleotide primer for amplifying a portion of efp gene.
SEQ ID NO: 11: Designed oligonucleotide primer for amplifying a portion of c-Myc-1 gene.
SEQ ID NO: 12: Designed oligonucleotide primer for amplifying a portion of c-Myc-1 gene.
SEQ ID NO: 13: Designed oligonucleotide primer for amplifying a portion of vitamin D receptor gene.
SEQ ID NO: 14: Designed oligonucleotide primer for amplifying a portion of vitamin D receptor gene.
SEQ ID NO: 15: Designed oligonucleotide primer for amplifying a portion of c-Myc-2 gene.
SEQ ID NO: 16: Designed oligonucleotide primer for amplifying a portion of c-Myc-2 gene.
SEQ ID NO: 17: Designed oligonucleotide primer for amplifying a portion of Bax gene.
SEQ ID NO: 18: Designed oligonucleotide primer for amplifying a portion of Bax gene.
SEQ ID NO: 19: Designed oligonucleotide primer for amplifying a portion of JNK1 gene.
SEQ ID NO: 20: Designed oligonucleotide primer for amplifying a portion of JNK1 gene.
SEQ ID NO: 21: Designed oligonucleotide primer for amplifying a portion of p38 gene.
SEQ ID NO: 22: Designed oligonucleotide primer for amplifying a portion of p38 gene.
SEQ ID NO: 23: Designed oligonucleotide primer for amplifying a portion of TRIP 1 gene.
SEQ ID NO: 24: Designed oligonucleotide primer for amplifying a portion;of TRIP 1 gene.
SEQ ID NO: 25: Designed oligonucleotide primer for amplifying a portion of ARA 70 gene.
SEQ ID NO: 26: Designed oligonucleotide primer for amplifying a portion of ARA 70 gene.
SEQ ID NO: 27: Designed oligonucleotide primer for amplifying a portion of insulin receptor gene.
SEQ ID NO: 28: Designed oligonucleotide primer for amplifying a portion of insulin receptor gene.
SEQ ID NO: 29: Designed oligonucleotide primer for amplifying a portion of PDGF receptor gene.
SEQ ID NO: 30: Designed oligonucleotide primer for amplifying a portions of PDGF receptor gene.
SEQ ID NO: 31: Designed oligonucleotide primer for amplifying a portion of CSF1 receptor-2 gene.
SEQ ID NO: 32: Designed oligonucleotide primer for amplifying a portion of CSF1 receptor-2 gene.
SEQ ID NO: 33: Designed oligonucleotide primer for amplifying a portion of FGF receptor gene.
SEQ ID NO: 34: Designed oligonucleotide primer for amplifying a portion of FGF receptor gene.
SEQ ID NO: 35: Designed oligonucleotide primer for amplifying a portion of p38 gamma gene.
SEQ ID NO: 36: Designed oligonucleotide primer for amplifying a portion of p38 gamma gene.
SEQ ID NO: 37: Designed oligonucleotide primer for amplifying a portion of Bcl-X gene.
SEQ ID NO: 38: Designed oligonucleotide primer for amplifying a portion of Bcl-X gene.
SEQ ID NO: 39: Designed oligonucleotide primer for amplifying a portion of c-Myc-3 gene.
SEQ ID NO: 40: Designed oligonucleotide primer for amplifying a portion of c-Myc-3 gene.
SEQ ID NO: 41: Designed oligonucleotide primer for amplifying a portion of pS2 protein gene.
SEQ ID NO: 42: Designed oligonucleotide primer for amplifying a portion of pS2 protein gene.
SEQ ID NO: 43: Designed oligonucleotide primer for amplifying a portion of lactoferrin gene.
SEQ ID NO: 44: Designed oligonucleotide primer for amplifying a portion of lactoferrin gene.
SEQ ID NO: 45: Designed oligonucleotide primer for amplifying a portion of RIP 140 gene.
SEQ ID NO: 46: Designed oligonucleotide primer for amplifying a portion of RIP 140 gene.
SEQ ID NO: 47: Designed oligonucleotide primer for amplifying a portion of TIF2 gene.
SEQ ID NO: 48: Designed oligonucleotide primer for amplifying a portion of TIF2 gene.
SEQ ID NO: 49: Designed oligonucleotide primer for amplifying a portion of JNK2 gene.
SEQ ID NO: 50: Designed oligonucleotide primer for amplifying a portion of JNK2 gene.
SEQ ID NO: 51: Designed oligonucleotide primer for amplifying a portion of Bax delta gene.
SEQ ID NO: 52: Designed oligonucleotide primer for amplifying a portion of Bax delta gene.
SEQ ID NO: 53: Designed oligonucleotide primer for amplifying a portions of BMK-1 gene.
SEQ ID NO: 54: Designed oligonucleotide primer for amplifying a portion of BMK-1 gene.
SEQ ID NO: 55: Designed oligonucleotide primer for amplifying a portion of BMK-2 gene.
SEQ ID NO: 56: Designed oligonucleotide primer for amplifying a portion of BMK-2 gene.
SEQ ID NO: 57: Designed oligonucleotide primer for amplifying a portion of Src-1 gene.
SEQ ID NO: 58: Designed oligonucleotide primer for amplifying a portion of Src-1 gene.
SEQ ID NO: 59: Designed oligonucleotide primer for amplifying a portion of p300/CBP gene.
SEQ ID NO: 60: Designed oligonucleotide primer for amplifying a portion of p300/CBP gene.
SEQ ID NO: 61: Designed oligonucleotide primer for amplifying a portion of β-actin gene.
SEQ ID NO: 62: Designed oligonucleotide primer for amplifying a portion of β-actin gene.

## Claims

1. A cord-, tape- or disk-shaped substrate having plural different ligands being immobilized in predetermined regions on its surface.

2. The substrate according to claim 1, wherein the ligands are nucleic acids, saccharides, peptides or proteins.

3. The substrate according to claim 2, wherein the nucleic acids are double-stranded DNAs or single-stranded DNAs.

4. The substrate according to claim 3, wherein the DNAs are polynucleotides or oligonucleotides.

5. The substrate according to any one of claims 1 to 4, which can be used to detect at high speed a binding between a ligand and a receptor having an ability to bind to the ligand.

6. The substrate according to claim 5, wherein the distance between the regions is at least 1 mm.

7. The substrate according to claim 5, wherein the density of the regions is less than 100 regions/cm².

8. The substrate according to claim 5, wherein the density of the regions is less than 10 regions/cm².

9. The substrate according to any one of claims 6 to 8, wherein the number of the regions on the substrate is 100 or more.

10. The substrate according to any one of claims 6 to 8, wherein the number of the regions on the substrate is 1000 or more.

11. The substrate according to any one of claims 5 to 10, wherein the receptor is a nucleic acid, a saccharide, a peptide or a protein.

12. The substrate according to claim 11, wherein the receptor is a receptor with a label.

13. An apparatus for detecting a binding between a ligand and a receptor, which comprises:
a) a cord-, tape- or disk-shaped substrate having plural different ligands being immobilized in predetermined regions on its surface;
b) a means of exposing the substrate to a receptor that binds to at least one of the ligands; and
c) a means of detecting a region of a binding between the receptor and the at least one of ligands on the substrate.

14. The apparatus according to claim 13, wherein the cord-, tape- or disk-shaped substrate is the cord-, tape- or disk-shaped substrate defined by any one of claims 2 to 12.

15. The apparatus according to claim 14, wherein the receptor is a receptor with a label.

16. The apparatus according to claim 15, wherein the region of a binding between the receptor and the at least one of ligands on the substrate is detected by means of the label.

17. The apparatus according to claim 15 or 16, wherein the receptor is a nucleic acid, a saccharide, a peptide or a protein.

18. An apparatus for detecting a binding between a ligand and a receptor, which comprises a means of detecting at high speed a region of a binding between a receptor and one of plural different ligands on a cord-, tape- or disk-shaped substrate having the ligands being immobilized in predetermined regions on its surface.

19. The apparatus according to claim 16, wherein the cord-, tape- or disk-shaped substrate is the cord-, tape- or disk-shaped substrate defined by any one of claims 2 to 12.

20. The apparatus according to claim 19, wherein the receptor is a receptor with a label.

21. The apparatus according to claim 20, wherein the region a binding between the receptor and the ligand on the substrate is detected by means of the label.

22. The apparatus according to claim 20 or 21, wherein the receptor is a nucleic acid, a saccharide, a peptide or a protein.

23. A method for detecting a binding between a ligand and a receptor, the method comprising:
a) exposing a cord-, tape- or disk-shaped substrate having plural different ligands being immobilized in predetermined regions on its surface to a receptor that binds to at least one of the ligands; and
b) detecting a region of a binding between the receptor and the at least one of ligands on the substrate.

24. The method according to claim 23, wherein the cord-, tape- or disk-shaped substrate is the cord-, tape- or disk-shaped substrate defined by any one of claims 2 to 12.

25. The method according to claim 23 or 24, wherein the region of a binding between the receptor and the ligand on the substrate is detected at high speed.

26. The method according to claim 25, wherein the receptor is a receptor with a label.

27. The method according to claim 26, wherein the region of a binding between the receptor and the ligand on the substrate is detected by means of the label.

28. The method according to claim 26 or 27, wherein the receptor is a nucleic acid, a saccharide, a peptide or a protein.


Sequence Listing

<110> Takara Shuzo Co., Ltd.

<120> Bases having ligands immobilized thereon

<130> 38-47

<140> EP 00970022.0
<141> 2000-10-24

<150> JP 11-315610
<151> 1999-11-05

<160> 62

<210> 1
<211> 19
<212> DNA
<213> Artificial Sequence

<220>
<223> Designed oligonucleotide primer for amplifying a portion of Smad3
gene.

<400> 1
caggtgtccc atcggaagg                                        19

<210> 2

<211> 22

<212> DNA

<213> Artificial Sequence

<220>

<223> Designed oligonucleotide primer for amplifying a portion of Smad3

gene.

<400> 2

ctctctggta gtggtaggga tt                                          22

<210> 3

<211> 20

<212> DNA

<213> Artificial Sequence

<220>

<223> Designed oligonucleotide primer for amplifying a portion of VEGF

receptor gene.

<400> 3

tacaagatcg acgttagctc                                             20

<210> 4

<211> 20

<212> DNA

<213> Artificial Sequence

<220>

<223> Designed oligonucleotide primer for amplifying a portion of VEGF

receptor gene.

<400> 4

cagccaaatt cacagttaaa                                    20

<210> 5

<211> 24

<212> DNA

<213> Artificial Sequence

<220>

<223> Designed oligonucleotide primer for amplifying a portion of ACTR

gene.

<400> 5

gctttgaaga tataatccga aggt                               24

<210> 6

<211> 25

<212> DNA

<213> Artificial Sequence

<220>

<223> Designed oligonucleotide primer for amplifying a portion of ACTR

gene.

<400> 6

ggcctggtga tgacagagta gataa                              25

<210> 7

<211> 24

<212> DNA

<213> Artificial Sequence

<220>

<223> Designed oligonucleotide primer for amplifying a portion of N-

CoR/SMRT gene.

<400> 7

tatggaggac cctatgaaag tgta                                    24

<210> 8

<211> 25

<212> DNA

<213> Artificial Sequence

<220>

<223> Designed oligonucleotide primer for amplifying a portion of N-

CoR/SMRT gene.

<400> 8

ttacgaccat gttctactag acctt                                   25

<210> 9

<211> 20

<212> DNA

<213> Artificial Sequence

<220>

<223> Designed oligonucleotide primer for amplifying a portion of efp

gene.

<400> 9

cgccgtgaag acgtgcttgg                                                20

<210> 10

<211> 25

<212> DNA

<213> Artificial Sequence

<220>

<223> Designed oligonucleotide primer for amplifying a portion of efp

gene.

<400> 10

tcttggtcag gctctgttca atctc                                          25

<210> 11

<211> 16

<212> DNA

<213> Artificial Sequence

<220>

<223> Designed oligonucleotide primer for amplifying a portion of c-

Myc-1 gene.

<400> 11

cgccaagctc gtctca                                                16

<210> 12

<211> 20

<212> DNA

<213> Artificial Sequence

<220>

<223> Designed oligonucleotide primer for amplifying a portion of c-

Myc-1 gene.

<400> 12

tcaactgttc tcgtcgtttc                                            20

<210> 13

<211> 21

<212> DNA

<213> Artificial Sequence

<220>

<223> Designed oligonucleotide primer for amplifying a portion of

vitamin D receptor gene.

<400> 13

caaacgctgt gtggacatcg g                                          21

<210> 14

<211> 23

<212> DNA

<213> Artificial Sequence

<220>

<223> Designed oligonucleotide primer for amplifying a portion of

vitamin D receptor gene.

<400> 14

ttctggatca tcttggcata gag                                    23

<210> 15

<211> 20

<212> DNA

<213> Artificial Sequence

<220>

<223> Designed oligonucleotide primer for amplifying a portion of c-

Myc-2 gene.

<400> 15

gtagtaattc cagcgagagg                                        20

<210> 16

<211> 19

<212> DNA

<213> Artificial Sequence

<220>

<223> Designed oligonucleotide primer for amplifying a portion of c-

Myc-2 gene.

<400> 16
ctatgggcaa agtttcgtg                                    19

<210> 17
<211> 20
<212> DNA
<213> Artificial Sequence

<220>
<223> Designed oligonucleotide primer for amplifying a portion of Bax
gene.

<400> 17
tgttttctga cggcaacttc                                   20

<210> 18
<211> 17
<212> DNA
<213> Artificial Sequence

<220>
<223> Designed oligonucleotide primer for amplifying a portion of Bax
gene.

<400> 18
gagcactccc gccacaa                                      17

<210> 19

<211> 19

<212> DNA

<213> Artificial Sequence

<220>

<223> Designed oligonucleotide primer for amplifying a portion of JNK1

gene.

<400> 19

gagcagaagc aagcgtgac                                                19

<210> 20

<211> 20

<212> DNA

<213> Artificial Sequence

<220>

<223> Designed oligonucleotide primer for amplifying a portion of JNK1

gene.

<400> 20

gacattgatg tacgggtgtt                                               20

<210> 21

<211> 17

<212> DNA

<213> Artificial Sequence

<220>

<223> Designed oligonucleotide primer for amplifying a portion of p38

gene.

<400> 21

gtgcccgagc gttacca                                                17

<210> 22

<211> 20

<212> DNA

<213> Artificial Sequence

<220>

<223> Designed oligonucleotide primer for amplifying a portion of p38

gene.

<400> 22

aaagttcatc ttcggcatct                                             20

<210> 23

<211> 20

<212> DNA

<213> Artificial Sequence

<220>

<223> Designed oligonucleotide primer for amplifying a portion of TRIP

1 gene.

<400> 23

aaatgctaaa gttcgcctat 20

<210> 24

<211> 18

<212> DNA

<213> Artificial Sequence

<220>

<223> Designed oligonucleotide primer for amplifying a portion of TRIP

1 gene.

<400> 24

acatggactc gccgttct 18

<210> 25

<211> 18

<212> DNA

<213> Artificial Sequence

<220>

<223> Designed oligonucleotide primer for amplifying a portion of ARA

70 gene.

<400> 25

agttgcataa gccgtcac 18

<210> 26

<211> 20

<212> DNA

<213> Artificial Sequence

<220>

<223> Designed oligonucleotide primer for amplifying a portion of ARA

70 gene.

<400> 26

actagccaat ctgataggtc                                        20

<210> 27

<211> 20

<212> DNA

<213> Artificial Sequence

<220>

<223> Designed oligonucleotide primer for amplifying a portion of

insulin receptor gene.

<400> 27

gctgccacca atacgtcatt                                        20

<210> 28

<211> 19

<212> DNA

<213> Artificial Sequence

<220>

<223> Designed oligonucleotide primer for amplifying a portion of

insulin receptor gene.

<400> 28

gcatcctgcc catcgaact                                              19

<210> 29

<211> 25

<212> DNA

<213> Artificial Sequence

<220>

<223> Designed oligonucleotide primer for amplifying a portion of PDGF

receptor gene.

<400> 29

tcaccattcc atgccgagta acaga                                       25

<210> 30

<211> 22

<212> DNA

<213> Artificial Sequence

<220>

<223> Designed oligonucleotide primer for amplifying a portion of PDGF

receptor gene.

<400> 30

aggacagtgg gcggtgggta gg                                          22

<210> 31

<211> 20

<212> DNA

<213> Artificial Sequence

<220>

<223> Designed oligonucleotide primer for amplifying a portion of CSF1

receptor-2 gene.

<400> 31

ccaccctgaa tgaagtcaac                                    20

<210> 32

<211> 20

<212> DNA

<213> Artificial Sequence

<220>

<223> Designed oligonucleotide primer for amplifying a portion of CSF1

receptor-2 gene.

<400> 32

ggtggatgga ttaagactga                                    20

<210> 33

<211> 20

<212> DNA

<213> Artificial Sequence

<220>

<223> Designed oligonucleotide primer for amplifying a portion of FGF

receptor gene.

<400> 33

cagactccgg cctctatgct                                                20

<210> 34

<211> 20

<212> DNA

<213> Artificial Sequence

<220>

<223> Designed oligonucleotide primer for amplifying a portion of FGF

receptor gene.

<400> 34

gggcttccag aacggtcaac                                                20

<210> 35

<211> 23

<212> DNA

<213> Artificial Sequence

<220>

<223> Designed oligonucleotide primer for amplifying a portion of p38

gamma gene.

<400> 35

tgatcgggct gctggacgta ttc                                            23

<210> 36

<211> 25

<212> DNA

<213> Artificial Sequence

<220>

<223> Designed oligonucleotide primer for amplifying a portion of p38

gamma gene.

<400> 36

agagggcttg cattggtcag gatag                                          25

<210> 37

<211> 22

<212> DNA

<213> Artificial Sequence

<220>

<223> Designed oligonucleotide primer for amplifying a portion of Bcl-X

gene.

<400> 37

ccgggagctg gtggttgact tt                                             22

<210> 38

<211> 21

<212> DNA

<213> Artificial Sequence

<220>

<223> Designed oligonucleotide primer for amplifying a portion of Bcl-X

gene.

<400> 38

ttcttaccca gccgccgttc t                                              21

<210> 39

<211> 20

<212> DNA

<213> Artificial Sequence

<220>

<223> Designed oligonucleotide primer for amplifying a portion of c-

Myc-3 gene.

<400> 39

gtagtaattc cagcgagagg                                                20

<210> 40

<211> 19

<212> DNA

<213> Artificial Sequence

<220>

<223> Designed oligonucleotide primer for amplifying a portion of c-

Myc-3 gene.

<400> 40

ctatgggcaa agtttcgtg                                    19

<210> 41

<211> 20

<212> DNA

<213> Artificial Sequence

<220>

<223> Designed oligonucleotide primer for amplifying a portion of pS2

protein gene.

<400> 41

ggcccagaca gagacgtgta                                   20

<210> 42

<211> 22

<212> DNA

<213> Artificial Sequence

<220>

<223> Designed oligonucleotide primer for amplifying a portion of pS2

protein gene.

<400> 42

gctcgatggt attaggatag aa                                22

<210> 43

<211> 19

<212> DNA

<213> Artificial Sequence

<220>

<223> Designed oligonucleotide primer for amplifying a portion of

lactoferrin gene.

<400> 43

ggagctgcgc aagtgtaac                                    19

<210> 44

<211> 20

<212> DNA

<213> Artificial Sequence

<220>

<223> Designed oligonucleotide primer for amplifying a portion of

lactoferrin gene.

<400> 44

attagtaatg cctgcgacat                                   20

<210> 45

<211> 20

<212> DNA

<213> Artificial Sequence

<220>

<223> Designed oligonucleotide primer for amplifying a portion of RIP

140 gene.

<400> 45
gcctctttgc ttcagtcatt                                    20

<210> 46
<211> 20
<212> DNA
<213> Artificial Sequence

<220>
<223> Designed oligonucleotide primer for amplifying a portion of RIP
140 gene.

<400> 46
ttggcttagg tatagtctgg                                    20

<210> 47
<211> 20
<212> DNA
<213> Artificial Sequence

<220>
<223> Designed oligonucleotide primer for amplifying a portion of TIF2
gene.

<400> 47
tccaaggcaa gatcacgtct                                    20

<210> 48

<211> 20

<212> DNA

<213> Artificial Sequence

<220>

<223> Designed oligonucleotide primer for amplifying a portion of TIF2

gene.

<400> 48

aagccaacga tgaccctaat                                                20

<210> 49

<211> 25

<212> DNA

<213> Artificial Sequence

<220>

<223> Designed oligonucleotide primer for amplifying a portion of JNK2

gene.

<400> 49

tatagtgtcc aagtggcaga ctcaa                                          25

<210> 50

<211> 23

<212> DNA

<213> Artificial Sequence

<220>

<223> Designed oligonucleotide primer for amplifying a portion of JNK2

gene.

<400> 50

atgtatgggt gacgcagagc ttc                                          23

<210> 51

<211> 24

<212> DNA

<213> Artificial Sequence

<220>

<223> Designed oligonucleotide primer for amplifying a portion of Bax

delta gene.

<400> 51

gatgattgcc gccgtggaca caga                                         24

<210> 52

<211> 25

<212> DNA

<213> Artificial Sequence

<220>

<223> Designed oligonucleotide primer for amplifying a portion of Bax

delta gene.

<400> 52

ggtgagcact cccgccacaa agatg                                25

<210> 53

<211> 25

<212> DNA

<213> Artificial Sequence

<220>

<223> Designed oligonucleotide primer for amplifying a portion of BMK-1

gene.

<400> 53

ttaaagcccg ctccttcgat gtgac                                25

<210> 54

<211> 23

<212> DNA

<213> Artificial Sequence

<220>

<223> Designed oligonucleotide primer for amplifying a portion of BMK-1

gene.

<400> 54

ggcggtcggc acctgggtac act                                  23

<210> 55

<211> 25

<212> DNA

<213> Artificial Sequence

<220>
<223> Designed oligonucleotide primer for amplifying a portion of BMK-2
gene.

<400> 55
ggtggccatc aagaagatcc ctaat                                    25

<210> 56
<211> 24
<212> DNA
<213> Artificial Sequence

<220>
<223> Designed oligonucleotide primer for amplifying a portion of BMK-2
gene.

<400> 56
cctcacgcct tgcatggaag tcct                                     24

<210> 57
<211> 25
<212> DNA
<213> Artificial Sequence

<220>
<223> Designed oligonucleotide primer for amplifying a portion of Src-1
gene.

<400> 57

gtatgaatga aggacccaat aactc                                    25

<210> 58

<211> 21

<212> DNA

<213> Artificial Sequence

<220>

<223> Designed oligonucleotide primer for amplifying a portion of Src-1

gene.

<400> 58

ctggcaggat ctccgatttg a                                        21

<210> 59

<211> 25

<212> DNA

<213> Artificial Sequence

<220>

<223> Designed oligonucleotide primer for amplifying a portion of

p300/CBP gene.

<400> 59

ttcagtcacc aacgtgccaa atatg                                    25

<210> 60

<211> 23

<212> DNA

<213> Artificial Sequence

<220>

<223> Designed oligonucleotide primer for amplifying a portion of

p300/CBP gene.

<400> 60

ttagagctgc gggatcaggt gtt                                    23

<210> 61

<211> 21

<212> DNA

<213> Artificial Sequence

<220>

<223> Designed oligonucleotide primer for amplifying a portion of -

actin gene.

<400> 61

caagagatgg ccacggctgc t                                      21

<210> 62

<211> 21

<212> DNA

<213> Artificial Sequence

<220>

<223> Designed oligonucleotide primer for amplifying a portion of -
actin gene.

<400> 62
tccttctgca tcctgtcggc a                                          21